# FASCICULE DE BREVET EUROPEEN

(11) **EP 2 435 101 B1**
(45) Date de publication et mention de la délivrance du brevet: **20.02.2013**
(21) Numéro de dépôt: 10728760.9
(22) Date de dépôt: 14.05.2010
(51) Int. Cl.: A61K 8/73, A61Q 19/08, A61K 8/04, A61K 8/00, A61L 27/52, A61L 27/20

(54) **HYDROGEL INJECTABLE PERMETTANT UNE SUPPLEMENTATION EN GLYCEROL DANS LA PEAU SUR LE LONG TERME**
INJIZIERBARES HYDROGEL FÜR DIE LANGZEITERGÄNZUNG DER HAUT MIT GLYCERIN
INJECTABLE HYDROGEL FOR THE LONG-TERM SUPPLEMENTATION OF GLYCEROL IN THE SKIN

(30) Priorité: 26.05.2009 FR 0953452
(43) Date de publication de la demande: 04.04.2012
(73) Titulaire: ANTEIS S.A., 1228 Plan-Les-Ouates, Genève (CH)
(72) Inventeur: GAVARD MOLLIARD, Samuel, 74250 Bogève (FR); VINCHON, Cyrille, 74270 Chilly (FR)
(74) Mandataire: Lienard, Céline
(86) Numéro de dépôt international: PCT/FR2010/050937
(87) Numéro de publication internationale: WO 2010/136694

(56) Documents cités:
- WO-A1-2008/068297
- FR-A1- 2 900 575
- FR-A1- 2 918 276

## Description

La présente invention se rapporte à un hydrogel injectable permettant une supplémentation en glycérol dans la peau sur le long terme.

La présente invention se rapporte également au procédé et à l'utilisation dans le domaine de la dermatologie de l'hydrogel susmentionné.

Le glycérol, molécule endogène, est un trihydroxy alcool ayant un rôle clé dans la peau. Le glycérol endogène joue notamment un rôle majeur dans l'hydratation de la peau, dans l'élasticité cutanée et dans la réparation de la barrière épidermique *(*Journal Britannique de Dermatologie, 159(1):13-34, juillet 2008, Fluhr, J. W.; Darlenski, R. **; Surber).*

Les actions bénéfiques diverses du glycérol sur l'épiderme incluent notamment l'hydratation du *stratum corneum* (couche la plus superficielle de la peau), la fonction barrière de la peau, les propriétés mécaniques de la peau, la protection contre les stimuli irritants et l'accélération du processus de cicatrisation de plaies *(*Journal Britannique de Dermatologie, 159(1):.23-34, juillet 2008, Fluhr, J. W.; Darlenski, R. **; Surber).*

D'après l'état de la technique, on sait également que l'application topique de produits contenant du glycérol améliore les propriétés de la peau dans les maladies caractérisées par du xérosis et par une barrière épidermique détériorée, comme c'est le cas dans la dermatite atopique.

De plus, avec l'âge, on observe une diminution de la quantité de glycérol dans la peau.

Sur la base de l'ensemble de ces éléments, on déduit qu'une supplémentation en glycérol est bénéfique pour la peau.

C'est pourquoi de nombreux cosmétiques possèdent du glycérol dans leur composition. Ainsi, par une application une à deux fois par jour, la peau bénéficie des effets positifs du glycérol. Toutefois, cette solution implique un traitement topique quotidien de la peau.

Tout comme l'utilisation d'un cosmétique, l'injection dans la peau d'une solution aqueuse de glycérol permet de bénéficier des effets positifs du glycérol sur le très court terme. En effet, la quasi-totalité du glycérol injecté migre rapidement de la zone d'injection vers le *stratum corneum,* puis est éliminée de la surface de la peau par lavage quotidien. Ainsi, l'action bénéfique du glycérol n'est que ponctuelle *(*PNAS, 100(11): 7360-7365, juin 2003*,* Hara, M.W.; Verkman, A.S. / Journal d'Investigation Dermatologique, 125: 288-293, 2005*, Choi, H.C).*

Des solutions injectables de glycérol contenant de l'acide hyaluronique (HA) sont décrites dans l'art antérieur. Ces solutions, de par leurs caractéristiques viscoélastiques, semblent permettre une libération retardée du glycérol. Toutefois, injectées dans la peau, ces solutions ne permettent pas une supplémentation du glycérol sur le long terme (supplémentation sur une période d'au maximum une semaine). En effet, l'acide hyaluronique est métabolisé au sein de la peau en moins d'une semaine *(Wenner-Gren International series; The chemistry, biology and medical applications of hyaluronan and its derivatives, Laurent, T.C.).*

Le brevet FR 2900575 décrit un gel injectable à libération contrôlé de principes actifs, le gel est constitué de hyaluronane et comprend du glycérol ainsi qu'un agent actif (revendication 4). Le taux de réticulation du gel n'est pas uniforme permettant ainsi une libération contrôlé du principe actif.

Par conséquent, il n'existe pas dans l'état de la technique un hydrogel injectable permettant une libération progressive et à long terme de glycérol dans la peau.

L'invention a ainsi pour but de proposer un nouvel hydrogel injectable qui présente de nombreuses qualités et qui évite une partie des inconvénients susmentionnés.

A cet effet, l'invention concerne un hydrogel injectable comprenant, en poids, dans un fluide vecteur physiologiquement acceptable : 0,01% à 5% de glycérol et 0,1% à 5% d'un biopolymère d'acide hyaluronique réticulé ou d'un de ses sels, par rapport au poids total de l'hydrogel, caractérisé en ce que l'acide hyaluronique ou l'un de ses sels est réticulé par formation de liaisons covalentes entre les chaînes dudit biopolymère à l'aide de molécules bi- ou polyfonctionnelles, ledit hydrogel injectable étant stérilisé à la chaleur humide et présentant un Tanδ à la fréquence de 1 Hz inférieur ou égal à 1,10.

Le paramètre Tanδ est un paramètre rhéologique usuel qui se définit comme la tangente de l'angle de phase, cet angle de phase étant la différence de phase entre la contrainte et la déformation lors d'un test rhéologique oscillatoire. La tangente de l'angle de phase permet de caractériser les propriétés viscoélastiques d'un gel, notamment à base d'acide hyaluronique réticulé comme cela à été démontré dans la publication de Falcone (Journal of biomedical materials Research, part A, janvier 2008, Falcone, S.J.; Berg, R.A.*).* Pour un gel à base d'acide hyaluronique réticulé, le paramètre Tanδ est vraisemblablement à corréler avec la rémanence du gel dans la peau, c'est-à-dire sa présence au niveau du site d'injection.

De manière tout à fait surprenante, un hydrogel à base de 0,01% à 5% de glycérol et de 0,1% à 5% d'acide hyaluronique réticulé par formation de liaisons covalentes, suivie d'une stérilisation à la chaleur humide, hydrogel possédant des propriétés viscoélastiques telles que Tanδ à la fréquence de 1 Hz est inférieur ou égal à 1,10 permet :
- de considérablement augmenter la durée de libération du glycérol dans la peau par rapport à un gel à base de glycérol et d'acide hyaluronique non réticulé stérilisé à la chaleur humide (type de gel avec glycérol décrit dans l'art antérieur) (voir l'exemple comparatif 2)
- de significativement augmenter la durée de libération du glycérol dans la peau par rapport à un gel à base de glycérol et d'acide hyaluronique réticulé non stérilisé à la chaleur humide (voir l'exemple comparatif 1)
- de significativement augmenter la rémanence, c'est-à-dire la présence du gel au niveau du site d'injection, par rapport à un gel à base de glycérol et d'acide hyaluronique réticulé non stérilisé à la chaleur humide (voir l'exemple comparatif 1)

Cette quadruple sélection : réticulation covalente de l'acide hyaluronique à une concentration adaptée, présence de glycérol à une concentration adaptée, stérilisation à la chaleur humide à une température suffisamment élevée et obtention d'un gel possédant des propriétés viscoélastiques particulières permet d'obtenir un hydrogel injectable avec glycérol ayant une rémanence de plusieurs mois et une capacité de libération du glycérol dans la peau sur une longue période. Ainsi, de par la combinaison des caractéristiques particulières de l'hydrogel injectable selon l'invention, le glycérol est élué progressivement hors du gel sur une longue période, permettant ainsi à la peau de bénéficier d'une supplémentation en glycérol au cours du temps sur le long terme.

Selon l'invention, la concentration en glycérol est comprise entre 0,01% à 5% en poids, de préférence de 0,5% à 2,5% par rapport au poids total de l'hydrogel. Au delà de 5% en poids, l'hydrogel possède une osmolarité trop élevée, qui le rend inadapté pour une injection dans la peau.

Selon l'invention, la concentration en acide hyaluronique réticulé ou d'un de ses sels est comprise entre 0,1% à 5% en poids, de préférence entre 0,5% à 3% par rapport au poids total de l'hydrogel.

Selon l'invention, l'hydrogel possède un Tanδ à la fréquence de 1 Hz inférieur ou égal à 1,10. De préférence, l'hydrogel possède un Tanδ à la fréquence de 1 Hz inférieur ou égal à 0,80.

De préférence, la masse moléculaire de l'acide hyaluronique ou de l'un de ses sels (avant réticulation) est comprise entre 1000 Da et 10×10⁶ Da, de préférence entre 500000 et 4×10⁶ Da.

Selon l'invention, la réticulation se fait par des molécules bi- ou polyfonctionnelles choisies par exemple parmi les époxydes, les épihalohydrines et la divinylsulfone, sur de l'acide hyaluronique non réticulé ou déjà réticulé avec ou sans un ou plusieurs autres polysaccharides d'origine naturelle. Par exemple, le réticulant utilisé est le butanediol diglycidyl éther (BDDE) ou la divinylsulfone (DVS).

Selon un mode particulier de l'invention, le gel peut également comprendre d'autres polymères biocompatibles (comme des polysaccharides d'origine naturelle) et/ou d'autres substances pharmacologiquement actives (comme la lidocaïne, un anesthésique local) ou non pharmacologiquement actives (comme les vitamines ou les sels minéraux) ayant des effets positifs sur l'organisme ou sur l'hydrogel.

Selon l'invention, ledit hydrogel injectable est stérilisé à la chaleur humide.

Avantageusement, la stérilisation se fait à une température supérieure à 100°C. Préférentiellement, la stérilisation se fait à une température supérieure ou égale à 121°C.

Par exemple, un des cycles de stérilisation suivants peut être utilisé : 131°C pendant 1 min / 130°C pendant 3 min / 125°C pendant 7 min / 121°C pendant 20 min ou 121°C pendant 10 min.

La présente invention a également pour objet un procédé de fabrication d'un hydrogel injectable tel que décrit ci-dessus, comprenant les étapes consistant à :
a) préparer un premier mélange comprenant au moins 0.1 % à 5% en poids d'un biopolymère d'acide hyaluronique réticulé ou d'un de ses sels par formation de liaisons covalentes entre les chaînes dudit biopolymère à l'aide de molécules bi- ou polyfonctionnelles,
b) ajouter avant, au cours ou après la réticulation du biopolymère d'acide hyaluronique ou d'un de ses sels lors de l'étape a), 0,01% à 5% en poids de glycérol, de sorte à former un mélange homogène,
c) mettre le gel ainsi obtenu sous forme prête à l'emploi,
d) stériliser le produit à la chaleur humide.

Le glycérol peut en effet être ajouté dans le gel à base d'acide hyaluronique réticulé au cours de la fabrication du gel à base d'acide hyaluronique réticulé, c'est-à-dire à l'étape a) avant ou pendant réticulation du gel à base d'acide hyaluronique ou tout simplement à l'issu de l'étape a) : après la réticulation du biopolymère.

Lors de ce procédé de fabrication d'un hydrogel injectable, la température de stérilisation est supérieure à 100°C.

De préférence, la stérilisation se fait à une température supérieure ou égale à 121°C.

En particulier, lors du procédé de fabrication de l'hydrogel, la stérilisation se fait en suivant un des cycles suivants: 131°C pendant 1 min / 130°C pendant 3 min /125°C pendant 7 min / 121°C pendant 20 min ou 121°C pendant 10 min.

Le gel ainsi obtenu possède des caractéristiques rhéologiques telles que Tanδ à la fréquence de 1 Hz est inférieur ou égal à 1,10 et préférentiellement, inférieur ou égal à 0,80.

Un but de la présente invention concerne aussi un kit se présentant sous forme de seringue et contenant l'hydrogel injectable tel que décrit ci-dessus.

Selon une variante de réalisation du kit, celui-ci peut se présenter sous forme de fiole ou flacon contenant l'hydrogel injectable selon l'invention et apte à être prélevé à l'aide d'une seringue d'injection.

La présente invention se rapporte également à l'utilisation d'un hydrogel injectable selon l'invention ou du kit tel que décrit ci-dessus, pour améliorer les caractéristiques chimiques, physiques et mécaniques de la peau des mammifères.

En particulier, la présente invention se rapporte également à l'utilisation d'un hydrogel injectable susmentionné ou du kit tel que décrit ci-dessus, pour combler des rides et ridules et/ou créer du volume et/ou hydrater la peau et/ou relancer l'activité cellulaire épidermique et/ou maintenir des propriétés mécaniques de fermeté et d'élasticité de la peau et/ou maintenir la stimulation épidermique et dermique et/ou pour stimuler l'activité anti-oxydante du derme et/ou prévenir le vieillissement cutané et/ou accélérer le processus de cicatrisation des plaies.

L'invention sera mieux comprise et d'autres buts, détails, caractéristiques et avantages de celle-ci apparaîtront plus clairement à la lecture de la description suivante d'exemples de réalisation, en référence aux figures annexées dans lesquelles :

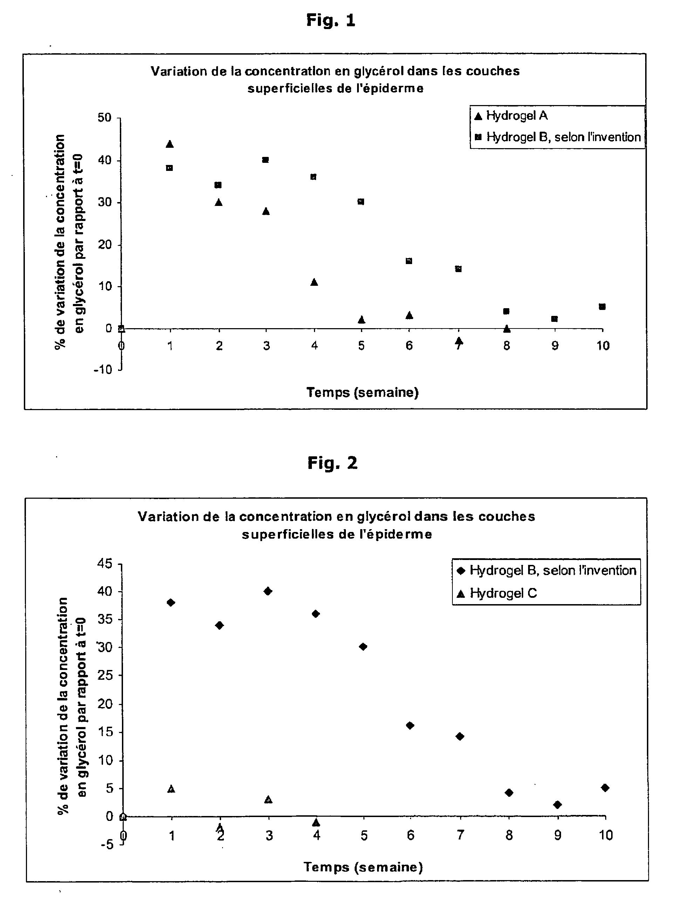

- la figure 1 représente une courbe montrant le pourcentage de variation de la concentration en glycérol dans les couches superficielles de l'épiderme de deux gels : gel A (gel n'ayant pas subi de stérilisation à la chaleur humide) et gel B (selon l'invention) au cours du temps en semaines ;
- de même, la figure 2 représente une courbe montrant le pourcentage de variation de la concentration en glycérol dans les couches superficielles de l'épiderme de deux gels : gel B (selon l'invention) et gel C (gel à base d'acide hyaluronique non réticulé) au cours du temps en semaines

### Exemple de réalisation :

Un exemple de préparation d'un hydrogel est proposé afin d'illustrer l'invention, mais en aucun cas il ne limite la portée de l'invention.

3,5 g de hyaluronate de sodium (MM=2,2.10⁶ Da) est ajouté dans 25 ml d'une solution de NaOH à 1 % (m/m). Le tout est laissé au repos pendant 1h puis mélangé à la spatule pendant 10 minutes. La réaction de réticulation est alors amorcée par ajout de 263 µl de butanediol diglycidyl éther (BDDE) et le tout est mélangé à la spatule pendant 10 minutes. Le mélange réactionnel est introduit dans un bain-marie à 50°C pendant 2h. Le mélange est réajusté au pH physiologique à l'aide d'HCl 1 M. Le volume est ajusté à 116 ml à l'aide d'une solution tamponnée à pH=7. Le gel ainsi obtenu est ensuite dialysé pendant 24h (cellulose régénérée, limite de séparation : MM=60kDa) contre une solution tamponnée à pH=7 afin d'éliminer le réticulant résiduel.

1,7% (m/m) de glycérol est ajouté dans le gel à base d'acide hyaluronique réticulé, puis le mélange est homogénéisé pendant 15 minutes à la spatule.

Le gel est ensuite introduit dans des seringues en verre de 1 ml.

La concentration totale en hyaluronate de sodium est mesurée à 2,1% (m/m).

La concentration totale en glycérol est mesurée à 1,68% (m/m).

Le pH du gel est mesuré à 7,02 et son osmolarité à 308 mOsm/kg.

La mesure de biocontamination initiale est inférieure à 10 CFU/g.

La moitié des seringues obtenues constituent l'hydrogel A.

L'autre moitié des seringues obtenues sont stérilisées à la chaleur humide suivant un cycle [131°C, 1 minute]. Ces seringues constituent l'hydrogel B, selon l'invention.

Les propriétés rhéologiques des hydrogels sont étudiées à l'aide d'un rhéomètre AR1000 (TA instruments) avec une géométrie plate de 40 mm, un entrefer de 1000 microns et une température d'analyse de 25°C.

Pour l'hydrogel A, la valeur mesurée du paramètre viscoélastique Tanδ (à 1 Hz) est égale à 0,51.

Pour l'hydrogel B, la valeur mesurée du paramètre viscoélastique Tanδ (à 1 Hz) est égale à 0,67.

### Essai comparatif 1 entre l'hydrogel A et l'hydrogel B (selon l'invention) de l'exemple ci-dessus :

Tests *in vivo* sur lapin : Sur un même lapin, quatre injections intradermiques calibrées de 0,2 ml de l'hydrogel A et quatre injections intradermiques calibrées de 0,2 ml de l'hydrogel B (hydrogel selon l'invention) ont été effectuées.

A t=0, on constate la présence d'une « papule » au niveau de chaque site d'injection, preuve de la présence du gel sur le site injecté. Le diamètre de chaque papule est mesuré à l'aide d'un pied à coulisse puis la surface moyenne initiale d'une papule est calculée pour l'hydrogel A et pour l'hydrogel B.

A t=3 mois, on constate toujours la présence de la totalité de ces papules pour les 2 types d'hydrogels injectés (les papules ont une surface moyenne supérieure au 1/2 de la surface moyenne initiale des 4 papules de l'hydrogel A ou des 4 papules de l'hydrogel B).

A t=6 mois, on constate que les papules formées avec l'hydrogel B selon l'invention sont encore présentes (les papules ont une surface moyenne supérieure au 1/2 de la surface moyenne initiale des 4 papules de l'hydrogel B) alors que les papules obtenues avec l'hydrogel A ne sont presque plus visibles (les papules ont une surface moyenne inférieure au 1/2 de la surface moyenne initiale des 4 papules de l'hydrogel A).

L'hydrogel B selon l'invention possède donc une rémanence significativement supérieure à celle de l'hydrogel A.

D'autre part, une mesure de la concentration en glycérol dans les couches superficielles de l'épiderme du lapin, au niveau de chaque papule obtenue, a été effectuée 1 fois par semaine pendant au maximum 10 semaines. Pour chaque gel étudié et pour chaque temps étudié, une moyenne des concentrations en glycérol dosées est calculée. Les évolutions de la concentration en glycérol dosée au cours du temps pour l'hydrogel A et pour l'hydrogel B sont comparées (voir figure 1)

On constate une cinétique de libération du glycérol dans la peau plus rapide pour l'hydrogel A que pour l'hydrogel B selon l'invention.

### Essai comparatif 2 entre l'hydrogel B (selon l'invention) de l'exemple ci-dessus et un hydrogel à base d'acide hyaluronique non réticulé et de glycérol, stérilisé à la chaleur humide (hydrogel C décrit ci-dessous):

Soit un gel constitué de 1,7% (m/m) de glycérol et de 2,1% (m/m) d'acide hyaluronique non réticulé (MM=2,2.10⁶ Da) dans une solution tamponnée à pH=7.
Après remplissage en seringues de verre de 1 ml, le gel est stérilisé à la chaleur humide suivant un cycle [131°C, 1 minute]. Ces seringues constituent l'hydrogel C.

Les propriétés rhéologiques de l'hydrogel C sont étudiées à l'aide d'un rhéomètre AR1000 (TA instruments) avec une géométrie plate de 40 mm, un entrefer de 1000 microns et une température d'analyse de 25°C.

Pour l'hydrogel C, la valeur mesurée du paramètre viscoélastique Tanδ (à 1 Hz) est égale à 0,64.

### Tests in vivo sur lapin :

Sur le même lapin que pour l'essai comparatif précédent, quatre injections intradermiques calibrées de 0,2 ml de l'hydrogel C ont été effectuées.

A t=0, on constate la présence d'une « papule » au niveau de chaque site d'injection, preuve de la présence du gel sur le site injecté. Le diamètre de chaque papule est mesuré à l'aide d'un pied à coulisse puis la surface moyenne initiale d'une papule est calculée pour l'hydrogel C.

A t=1 semaine, on constate que les papules obtenues avec l'hydrogel C ne sont presque plus visibles (les papules ont une surface moyenne inférieure au 1/2 de la surface moyenne initiale des 4 papules de l'hydrogel C).

A t=2 semaines, on constate que la totalité des papules obtenues avec l'hydrogel C ne sont plus visibles.

L'hydrogel B selon l'invention possède donc une rémanence considérablement supérieure à celle de l'hydrogel C.

D'autre part, une mesure de la concentration en glycérol dans les couches superficielles de l'épiderme du lapin, au niveau de chaque papule obtenue, a été effectuée après 1, 2, 3 et 4 semaines. Pour chaque temps étudié, une moyenne des concentrations en glycérol dosées est calculée. Les évolutions de la concentration en glycérol dosée au cours du temps pour l'hydrogel C et pour l'hydrogel B sont comparées (voir figure 2).

On constate une cinétique de libération du glycérol dans la peau considérablement plus rapide pour l'hydrogel C que pour l'hydrogel B selon l'invention.

On constate ainsi une véritable synergie entre l'acide hyaluronique réticulé et le glycérol, après stérilisation à la chaleur humide dans la formulation selon l'invention. La présence de glycérol dans la formulation selon l'invention permet d'augmenter significativement sa rémanence, c'est-à-dire le temps de résidence du gel au niveau du site d'injection, et donc par conséquent, permet d'augmenter également la libération du glycérol dans la peau au cours du temps.

Ainsi, le gel à base de glycérol et d'acide hyaluronique réticulé par formation de liaisons covalentes ayant subi une stérilisation à la chaleur humide et possédant des propriétés viscoélastiques telles que Tanδ à la fréquence de 1 Hz est inférieur ou égal à 1,10 permet une libération progressive et sur le long terme de glycérol dans la peau. Le glycérol et l'acide hyaluronique réticulé agissent en synergie lorsqu'ils sont associés selon la présente invention. Ceci peut s'expliquer par le fait que la stérilisation à la chaleur humide, dans les conditions de l'invention, permettrait une activation thermique du glycérol et/ou de l'acide hyaluronique réticulé, ce qui permettrait une meilleure affinité HA réticulé-glycérol et par conséquent, une libération sur le plus long terme du glycérol dans la peau (par rapport à un gel non stérilisé à la chaleur humide).

Il est important de préciser pour mettre clairement en évidence la pertinence de l'invention:
- qu'un gel à base d'acide hyaluronique (non réticulé) avec glycérol, stérilisé à la chaleur humide (type de gel décrit dans l'art antérieur), ne permet pas d'assurer une libération du glycérol dans la peau sur le long terme, sa durée de libération est en effet inférieure à environ 1 semaine ;
- qu'un gel à base d'acide hyaluronique réticulé de manière covalente avec glycérol, non stérilisé à la chaleur humide, ne permet pas d'obtenir des résultats équivalents à la formulation selon l'invention (durée de libération du glycérol dans la peau significativement plus courte).
- qu'un gel à base d'acide hyaluronique réticulé de manière covalente avec glycérol, possédant des propriétés rhéologiques telles que Tanδ à la fréquence de 1 Hz est supérieur à 1,10 ne possède pas un caractère viscoélastique adapté et/ou une rémanence suffisante pour obtenir des résultats équivalents à la formulation selon l'invention. La durée de libération du glycérol dans la peau est en effet significativement plus courte.

Il est également important de préciser que ces éléments ne pouvaient pas être déduits par l'homme de l'art.

En effet, l'homme de l'art sait que la réticulation d'un gel à base d'acide hyaluronique permet d'augmenter la rémanence de ce gel dans la peau. Toutefois, compte tenu de la faible masse moléculaire du glycérol (MM=92 g/mol) et de la faible concentration en acide hyaluronique dans le gel (concentration inférieure à 5% en poids), l'homme de l'art peut aisément supposer que la molécule de glycérol va facilement migrer au sein du gel (encombrement stérique du gel induit par l'acide hyaluronique insuffisant pour piéger le glycérol dans le gel) et par conséquent que le glycérol va rapidement être libéré dans la peau. L'homme de l'art peut également aisément supposer que la réticulation de l'acide hyaluronique va peu modifier la cinétique de libération du glycérol dans la peau par rapport à un gel à base d'acide hyaluronique non réticulé car le pontage des chaînes d'acide hyaluronique n'engendre pas un accroissement important de l'encombrement stérique au sein du gel.

En d'autres termes, l'homme de l'art ne pouvait pas présager que la combinaison [glycérol + acide hyaluronique réticulé par formation de liaisons covalentes + stérilisation à la chaleur humide + propriétés viscoélastiques particulières] permettrait une libération progressive du glycérol dans la peau sur le long terme.

Bien que l'invention ait été décrite en relation avec un mode particulier de réalisation, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Hydrogel injectable comprenant, en poids, dans un fluide vecteur physiologiquement acceptable : 0,01% à 5% de glycérol et 0,1% à 5% d'un biopolymère d'acide hyaluronique réticulé ou un de ses sels par rapport au poids total de l'hydrogel, **caractérisé en ce que** l'acide hyaluronique ou l'un de ses sels est réticulé par formation de liaisons covalentes entre les chaînes dudit biopolymère à l'aide de molécules bi- ou polyfonctionnelles, ledit hydrogel injectable étant stérilisé à la chaleur humide et présentant un Tanδ à la fréquence de 1 Hz inférieur ou égal à 1,10.

2. Hydrogel injectable selon la revendication 1, dans lequel le glycérol est présent à une concentration en poids allant de 0,5% à 2,5% par rapport au poids total de l'hydrogel.

3. Hydrogel injectable selon l'une des revendications précédentes, dans lequel l'acide hyaluronique ou l'un de ses sels est présent à une concentration en poids allant de 0,5% à 3% par rapport au poids total de l'hydrogel.

4. Hydrogel injectable selon l'une des revendications précédentes, pour lequel le paramètre Tanδ à la fréquence de 1 Hz est inférieur ou égal à 0,80.

5. Hydrogel injectable selon l'une des revendications précédentes, dans lequel la masse moléculaire de l'acide hyaluronique ou de l'un de ses sels est comprise entre 1000 Da et 10×10⁶ Da, de préférence entre 500000 et 4×10⁶ Da.

6. Hydrogel injectable selon l'une des revendications précédentes, dans lequel la réticulation se fait par des molécules bi- ou polyfonctionnelles choisies parmi les époxydes, les épihalohydrines et la divinylsulfone, sur de l'acide hyaluronique non réticulé ou déjà réticulé avec ou sans un ou plusieurs autres polysaccharides d'origine naturelle. Par exemple, le réticulant utilisé peut être le butanediol diglycidyl éther (BDDE) ou la divinylsulfone (DVS).

7. Hydrogel injectable selon l'une des revendications précédentes, **caractérisé en ce qu'**il comprend au moins un autre polymère biocompatible et/ou au moins une substance pharmacologiquement active et/ou au moins une substance non pharmacologiquement active ayant des effets positifs sur l'organisme ou sur l'hydrogel.

8. Procédé de fabrication d'un hydrogel injectable, comprenant les étapes consistant à :
a) préparer un premier mélange comprenant au moins 0.1 % à 5% en poids d'un biopolymère d'acide hyaluronique réticulé ou d'un de ses sels par formation de liaisons covalentes entre les chaînes dudit biopolymère à l'aide de molécules bi- ou polyfonctionnelles,
b) ajouter avant, au cours ou après la réticulation du biopolymère d'acide hyaluronique ou d'un de ses sels lors de l'étape a), 0,01% à 5% en poids de glycérol, de sorte à former un mélange homogène,
c) mettre le gel ainsi obtenu sous forme prête à l'emploi,
d) stériliser le produit à la chaleur humide.

9. Procédé de fabrication d'un hydrogel injectable selon la revendication 8, dans lequel la température de stérilisation est supérieure à 100°C.

10. Procédé de fabrication d'un hydrogel injectable selon l'une des revendications 8 à 9, dans lequel la stérilisation se fait à une température supérieure ou égale à 121°C.

11. Procédé de fabrication d'un hydrogel injectable selon l'une des revendications 8 à 10, dans lequel la stérilisation se fait en suivant un des cycles suivants: 131°C pendant 1 min / 130°C pendant 3 min / 125°C pendant 7 min / 121°C pendant 20 min ou 121°C pendant 10 min.

12. Procédé de fabrication d'un hydrogel injectable selon l'une des revendications 8 à 11, pour lequel le gel obtenu possède un Tanδ à la fréquence de 1 Hz inférieur ou égal à 1,10, préférentiellement inférieur ou égal à 0,80.

13. Kit se présentant sous forme de seringue et contenant l'hydrogel injectable selon l'une quelconque des revendications 1 à 7.

14. Kit se présentant sous forme de fiole ou flacon et contenant l'hydrogel injectable selon l'une quelconque des revendications 1 à 7 et apte à être prélevé à l'aide d'une seringue d'injection.

15. Hydrogel injectable selon l'une des revendications 1 à 7 ou kit selon l'une des revendications 13 à 14 appliqué pour améliorer les caractéristiques chimiques, physiques et mécaniques de la peau de mammifère.

16. Hydrogel injectable selon l'une des revendications 1 à 7 ou kit selon l'une des revendications 13 à 14 appliqué pour combler des rides et ridules et/ou créer du volume et/ou hydrater la peau et/ou relancer l'activité cellulaire épidermique et/ou maintenir des propriétés mécaniques de fermeté et d'élasticité de la peau et/ou maintenir la stimulation épidermique et dermique et/ou pour stimuler l'activité anti-oxydante du derme et/ou prévenir le vieillissement cutané et/ou accélérer le processus de cicatrisation des plaies.

## Claims

1. An injectable hydrogel comprising by weight in a physiologically acceptable fluid vector: 0.01 % to 5 % glycerol and 0.1 % to 5 % of a biopolymer of cross-linked hyaluronic acid or one of the salts thereof relative to the total weight of the hydrogel, **characterized in that** the hyaluronic acid or one of the salts thereof is cross-linked via the formation of covalent bonds between the chains of said biopolymer using bi- or polyfunctional molecules, said injectable hydrogel being sterilised under moist heat and having a Tanδ at a frequency of 1 Hz equal to or lower than 1.10.

2. The injectable hydrogel according to claim 1, wherein the glycerol is present in a weight concentration ranging from 0.5 % to 2.5 % relative to the total weight of the hydrogel.

3. The injectable hydrogel according to one of the preceding claims, wherein the hyaluronic acid or one of the salts therefore is present in a weight concentration ranging from 0.5 % to 3 % relative to the total weight of the hydrogel.

4. The injectable hydrogel according to one of the preceding claims, in which the Tanδ parameter, at the frequency of 1 Hz, is equal to or lower than 0.80.

5. The injectable hydrogel according to one of the preceding claims, wherein the molecular weight of the hyaluronic acid or one of the salts thereof is between 1000 Da and 10 x 10⁶ Da, preferably between 500000 and 4 x 10⁶ Da.

6. The injectable hydrogel according to one of the preceding claims, wherein cross-linking is performed using bi- or polyfunctional molecules chosen from among epoxides, epihalohydrins and divinylsulfone, on non-cross-linked hyaluronic acid or already cross-linked with or without one or more other polysaccharides of natural origin. For example, the cross-slinking agent used may be butanediol diglycidyl ether (BDDE) or divinylsulfone (DVS).

7. The injectable hydrogel according to one of the preceding claims, **characterized in that** it comprises at least one other biocompatible polymer and/or at least one pharmacologically active substance and/or at least one non-pharmacologically active substance having positive effects on the body or on the hydrogel.

8. A method for manufacturing an injectable hydrogel comprising the steps of:
a) preparing a first mixture comprising at least 0.1 % to 5
% by weight of a biopolymer of cross-linked hyaluronic acid or one of the salts thereof by forming covalent bonds between the chains of said biopolymer using bi- or polyfunctional molecules;
b) before, during or after the cross-linking at step a) of the biopolymer of hyaluronic acid or one of the salts thereof, adding 0.01 % to 5 % by weight of glycerol so as to form a homogeneous mixture,
c) placing the gel thus obtained in ready-to-use form,
d) sterilising the product under moist heat.

9. The method for manufacturing an injectable hydrogel according to claim 8, wherein the sterilisation temperature is higher than 100°C.

10. The method for manufacturing an injectable hydrogel according to one of claims 8 to 9, wherein sterilisation is performed at a temperature of 121 °C or higher.

11. The method for manufacturing an injectable hydrogel according to one of claims 8 to 10, wherein sterilisation is performed as per one of the following cycles: 131 °C for 1 min / 130°C for 3 min /125°C for 7 min / 121°C for 20 min or 121°C for 10 min.

12. The method for manufacturing an injectable hydrogel according to one of claims 8 to 11, in which the gel obtained has a Tanδ at the frequency of 1 Hz equal to or lower than 1.10, preferably equal to or lower than 0.80.

13. A kit in the form of a syringe containing the injectable hydrogel according to any of claims 1 to 7.

14. A kit in the form of a vial or bottle containing the injectable hydrogel according to any of claims 1 and 7 that is able to be drawn off by means of an injection syringe.

15. The injectable hydrogel according to one of claims 1 to 7, or the kit according to one of claims 13 to 14, that is applied to improve chemical, physical and mechanical characteristics of mammalian skin.

16. The injectable hydrogel according to one of claims 1 to 7, or the kit according to one of claims 13 to 14, that is applied to fill wrinkles or fine lines and/or to create volume and/or to hydrate the skin and/or to re-launch epidermal cellular activity and/or to maintain mechanical properties of skin firmness and elasticity and/or to maintain epidermal and dermal stimulation and/or to stimulate antoxidant activity of the dermis and/or to prevent skin ageing and/or to accelerate the process of wound healing.

## Patentansprüche

1. Injizierbares Hydrogel, das, in Gewicht, in einem physiologisch akzeptablen Vektorfluid umfasst: 0,01 % bis 5 % Glycerol und 0,1 % bis 5 % eines Biopolymers einer vernetzten Hyaluronsäure oder eines ihrer Salze im Verhältnis zum Gesamtgewicht des Hydrogels, **dadurch gekennzeichnet, dass** die Hyaluronsäure oder eines ihrer Salze durch Bildung kovalenter Bindungen zwischen den Ketten des Biopolymers mit Hilfe von Bi- oder Polyfunktionsmolekülen vernetzt ist, wobei das injizierbare Hydrogel bei feuchter Wärme sterilisiert ist und einen Tanδ mit der Frequenz 1 Hz kleiner oder gleich 1,10 aufweist.

2. Injizierbares Hydrogel nach Anspruch 1, wobei das Glycerol in einer Gewichtskonzentration von 0,5 % bis 2,5 % im Verhältnis zum Gesamtgewicht des Hydrogels vertreten ist.

3. Injizierbares Hydrogel nach einem der vorangehenden Ansprüche, wobei die Hyaluronsäure oder eines ihrer Salze in einer Gewichtskonzentration von 0,5 % bis 3 % im Verhältnis zum Gesamtgewicht des Hydrogels vertreten ist.

4. Injizierbares Hydrogel nach einem der vorangehenden Ansprüche, für das der Parameter Tanδ mit der Frequenz 1 Hz kleiner oder gleich 0,80 ist.

5. Injizierbares Hydrogel nach einem der vorangehenden Ansprüche, wobei die molekulare Masse der Hyaluronsäure oder eines ihrer Salze zwischen 1000 Da und 10x10⁶ Da, vorzugsweise zwischen 500000 und 4x10⁶ Da, inklusive ist.

6. Injizierbares Hydrogel nach einem der vorangehenden Ansprüche, wobei die Vernetzung durch Bi- oder Polyfunktionsmoleküle erfolgt, die aus den Epoxiden, den Epihalohydrinen und dem Divinylsulfon ausgewählt sind, auf mit oder ohne einen oder mehreren natürlichen Polysaccariden nicht vernetzter oder bereits vernetzter Hyaluronsäure. Das verwendete Vernetzungsmittel kann beispielsweise der Butanediol-Diglycidyl-Ether (BDDE) oder das Divinylsulfon (DVS) sein.

7. Injizierbares Hydrogel nach einem der vorangehenden Ansprüche, **dadurch gekennzeichnet, dass** es mindestens ein anderes biologisch kompatibles Polymer und/oder mindestens eine pharmakologisch aktive Substanz und/oder mindestens eine pharmakologisch nicht aktive Substanz umfasst, die positive Wirkungen auf den Organismus oder auf das Hydrogel hat.

8. Herstellungsverfahren eines injizierbaren Hydrogels, das die Schritte umfasst, die bestehen im:
a) Vorbereiten eines ersten Gemischs, das mindesten 0,1 bis 5 Gew.-% eines Biopolymers einer vernetzten Hyaluronsäure oder eines ihrer Salze durch Bildung kovalenter Bindungen zwischen den Ketten des Biopolymers mit Hilfe von Bi- oder Polyfunktionsmolekülen umfasst,
b) Hinzufügen vor, während und nach der Vernetzung des Biopolymers einer Hyaluronsäure oder eines ihrer Salze bei Schritt a) von 0,01 bis 5 Gew.-% Glycerol derart, dass ein homogenes Gemisch entsteht,
c) Ausgestalten des derart hergestellten Gels in eine gebrauchsfertige Form,
d) Sterilisieren des Produkts mit feuchter Wärme.

9. Herstellungsverfahren eines injizierbaren Hydrogels nach Anspruch 8, wobei die Sterilisationstemperatur höher als 100 °C ist.

10. Herstellungsverfahren eines injizierbaren Hydrogels nach einem der Ansprüche 8 bis 9, wobei die Sterilisation bei einer Temperatur über oder gleich 121 °C erfolgt.

11. Herstellungsverfahren eines injizierbaren Hydrogels nach einem der Ansprüche 8 bis 10, wobei die Sterilisation gemäß einem der folgenden Zyklen erfolgt: 131 °C während 1 Minute / 130 °C während 3 Minuten / 125 °C während 7 Minuten / 121 °C während 20 Minuten oder 121 °C während 10 Minuten.

12. Herstellungsverfahren eines injizierbaren Hydrogels nach einem der Ansprüche 8 bis 11, wobei das hergestellte Gel einen Tanδ mit der Frequenz 1 Hz kleiner oder gleich 1,10, vorzugsweise kleiner oder gleich 0,80, aufweist.

13. Kit in Spritzenform, ein injizierbares Hydrogel nach einem der Ansprüche 1 bis 7 enthaltend.

14. Kit in Phiolen- oder Flakonform, ein injizierbares Hydrogel nach einem der Ansprüche 1 bis 7 enthaltend und imstande, mit Hilfe einer Injektionsspritze entnommen zu werden.

15. Injizierbares Hydrogel nach einem der Ansprüche 1 bis 7 oder Kit nach einem der Ansprüche 13 bis 14, das angewendet wird, um die chemischen, physikalischen und mechanischen Merkmale der Haut eines Säugetiers zu verbessern.

16. Injizierbares Hydrogel nach einem der Ansprüche 1 bis 7 oder Kit nach einem der Ansprüche 13 bis 14, das angewendet wird, um Falten und Fältchen aufzupolstern und/oder Volumen zu bilden und/oder die Haut mit Feuchtigkeit zu versorgen und/oder die Zellaktivität der Epidermis anzukurbeln und/oder die mechanischen Straffheits- und Elastizitätseigenschaften der Haut aufrechtzuerhalten und/oder die Stimulation der Epidermis und Dermis aufrechtzuerhalten und/oder die antioxidative Aktivität der Dermis zu stimulieren und/oder der Hautalterung vorzubeugen und/oder den Prozess der Heilung von Wunden zu beschleunigen.
